# EUROPEAN PATENT SPECIFICATION

(11) **EP 4 111 463 B1**
(45) Date of publication and mention of the grant of the patent: **30.09.2026**
(21) Application number: 21760788.6
(22) Date of filing: 25.02.2021
(51) Int. Cl.: G16H 40/00, G16H 40/63, G16H 20/13, A61J 1/03, A61J 7/04

(54) **MEDICINE DISPENSER WITH PATIENT COMPLIANCE MONITORING SYSTEM**
MEDIKAMENTENSPENDER MIT PATIENTENKONFORMITÄTSÜBERWACHUNGSSYSTEM
DISTRIBUTEUR DE MÉDICAMENTS DOTÉ D'UN SYSTÈME DE SURVEILLANCE D'OBSERVANCE DE PATIENT

(30) Priority: 26.02.2020 US 202062982023 P
(43) Date of publication of application: 04.01.2023
(73) Proprietor: The Board of Regents of the University of Oklahoma, Norman, OK 73019 (US)
(72) Inventor: BUSINELLE, Michael, Oklahoma City, OK 73104 (US); FITZMORRIS, Cliff, Yukon, OK 73099 (US); WENG, BinBin, Norman, OK 73072 (US)
(74) Representative: J A Kemp LLP
(86) International application number: PCT/US2021/019721
(87) International publication number: WO 2021/173866

(56) References cited:
- US-A1- 2017 242 976
- US-A1- 2018 247 704
- US-A1- 2019 130 078
- US-A1- 2019 130 078
- US-A1- 2020 022 876
- US-A1- 2020 022 876

## Description

### BACKGROUND

Smoking is the leading cause of preventable death in the United States and is implicated in an estimated 30% of all cancer deaths in this country. Recent studies indicate that smokers have average life expectancies that are 11-12 years shorter than those who have never smoked. Importantly, depending on the age that one quits, ex-smokers live up to a decade longer than those who do not quit. Although the prevalence of smoking has declined over the past several decades, approximately 34 million (15% of) adults continue to smoke in the United States. Nearly 70% of smokers report that they want to quit smoking, and 52% of all smokers try to quit each year.

Many studies have indicated that the combination of behavioral support with pharmacotherapy provides optimal smoking cessation outcomes. However, only 25% of U.S. adult cigarette smokers reported using nicotine patches or oral nicotine replacement therapy (NRT) during their most recent quit attempt, and only 15% sought help from a doctor or other health professional.

Recently, research has focused on evaluating combination pharmacotherapies in an effort to refine current evidence-based treatment approaches. Nicotine replacement therapy comes in various forms including a skin patch, lozenges, and gum. The patch provides a steady nicotine dose whereas the lozenges and gum can provide acute relief during cravings and high-risk situations. The lozenges offer individuals the flexibility to deliver nicotine when they need it most, in contrast with the continuous and passive delivery of nicotine offered via the patch. While NRT monotherapy has been shown to result in greater smoking cessation success rates than no cessation medication, combination NRT (e.g., patch + oral NRT) significantly outperforms NRT monotherapy. Unfortunately, most smokers who use oral NRT do not use it as directed and discontinue use prematurely, increasing the likelihood of smoking lapse. It is difficult to accurately monitor patient compliance with treatment protocols using conventional patient compliance surveys. US 2017/242976 discloses background art to the invention.

Intelligent medication reminders that only prompt oral NRT use when a dose is missed could increase adherence and reduce the incidence of smoking lapse. Recent studies have shown that targeted alerts increase both acceptability and effectiveness, and increase medication adherence. There is, therefore, a need for an improved system and process for improving patient compliance with treatment protocols involving the periodic ingestion of oral medications, including, but not limited to, the oral ingestion of NRT lozenges. The present disclosure is directed to addressing these and other deficiencies in the prior art.

### SUMMARY

The invention is defined by the independent claims. Further aspects and preferred embodiments are defined in the dependent claims. Any aspects, embodiments and examples of the present disclosure which do not fall under the scope of the appended claims do not form part of the invention and are merely provided for illustrative purposes.

### BRIEF DESCRIPTION OF THE DRAWINGS

FIG. 1 depicts an oral medication compliance system that includes a pill dispenser constructed in accordance with an exemplary embodiment.
FIG. 2 is a top view of the pill dispenser of FIG. 1.
FIG. 3 is a bottom perspective view of the pill dispenser of FIG. 1.
FIG. 4 is a cutaway view of the pill dispenser of FIG. 2 illustrating internal components of the pill dispenser.
FIG. 5 is a top perspective view of a two-part pill dispenser constructed in accordance with a first embodiment.
FIG. 6 is a top perspective view of the pill dispenser of FIG. 5 with the two parts separated.
FIG. 7 is a cutaway, top view of a two-part pill dispenser constructed in accordance with a second embodiment.
FIG. 8 is a side view of a first half of the two-part pill dispenser of FIG. 7.
FIG. 9 is a side view of a second half of the two-part pill dispenser of FIG. 7.

### DETAILED DESCRIPTION

Tracking patient compliance with treatment protocols is an important aspect of delivering high quality healthcare. In one aspect, the present disclosure is directed, in non-limiting embodiments, to a system and method for improving compliance and monitoring of a treatment protocol that includes the periodic ingestion of oral medications. The system includes a mobile computing device and a pill tracking dispenser. The mobile computing device includes an application program configured to record the periodic consumption of oral medication by the patient. The pill tracking dispenser can be connected to the mobile computing device through a wireless network. The pill tracking dispenser is configured to report to the mobile computing device the number of times the oral medication is consumed by the patient.

In another aspect, the present disclosure is directed to a pill tracking dispenser that includes a pill chamber configured to contain a plurality of pills, a pill release mechanism, an electronics chamber that includes an onboard processing unit, and a gate sensor. The pill release mechanism includes a gate that permits a pill to be released from the pill chamber when the gate is opened. The onboard processing unit is configured to establish a data connection with an oral medication compliance system that includes a mobile computing device that is in turn configured for connection to a computer system. The gate position sensor is configured to detect the gate opening and provide the onboard processing unit with a signal that the gate was opened to permit the release of a pill.

In yet another aspect, the present disclosure is directed to a pill tracking dispenser configured for use within a system for tracking patient compliance with a treatment protocol that includes the periodic consumption of oral medication. In these embodiments, the pill tracking dispenser includes a pill chamber configured to contain a plurality of pills and a pill release mechanism that includes a gate that permits a pill to be released from the pill chamber when the gate is opened. The pill tracking dispenser also includes a trigger connected to the gate, where the trigger can be manipulated by the patient to move the gate into an open position. The pill tracking dispenser 102 further includes an onboard processing unit that is configured to establish a data connection with a mobile computing device that is in turn configured for connection to a computer system used by a clinician in monitoring the patient's compliance with the treatment protocol.

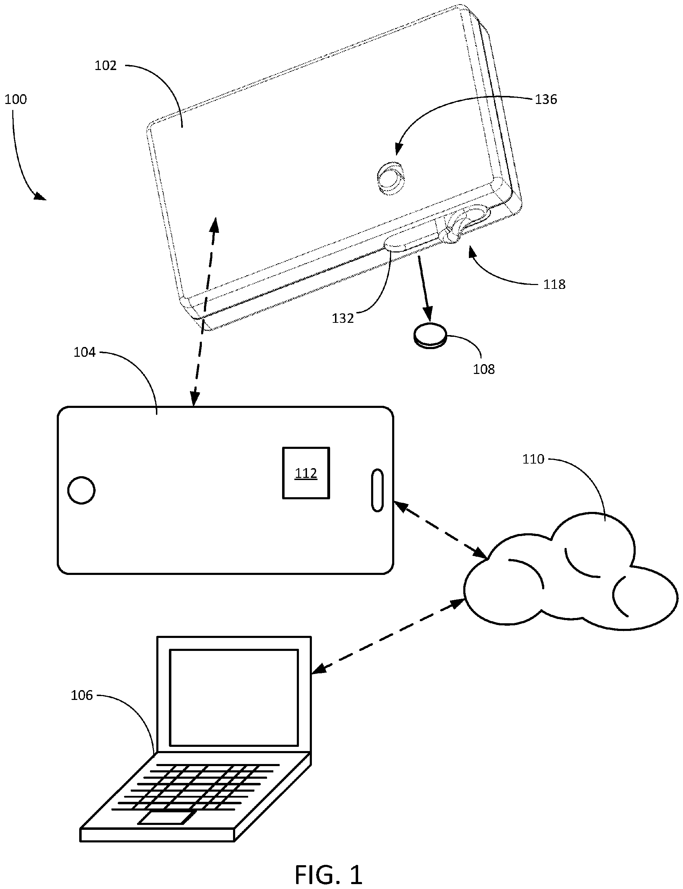

Beginning with FIG. 1, shown therein is a simplified depiction of an oral medication compliance system 100 that includes a pill tracking dispenser 102, a mobile computing device 104 and a computer system 106 used by a clinician, physician or study administrator. The pill tracking dispenser 102 is configured to track the dispensation of an oral medication 108 in the form of pills, lozenges, tablets, capsules or other solid forms of medication (referred to generically as "pills" in this disclosure). Generally, the pill tracking dispenser 102 is configured to detect and record each pill release event and the time that each pill release event occurred. The pill tracking dispenser 102 provides this information to the computer system 106 through a direct data connection or through an intermediate data network 110, such as the internet, that is accessed through the mobile computing device 104.

In exemplary embodiments, the mobile computing device 104 is a smartphone or tablet that is configured to be connected to the pill tracking dispenser 102 with a paired wireless data connection, such as through a Bluetooth or Wi-Fi connection protocol. In some embodiments, the pill tracking dispenser 102 and mobile computing device 104 are configured to exchange data each time a connection is available between the two devices. In other embodiments, the pill tracking dispenser 102 and mobile computing device 104 exchange data on a delayed, periodic basis (e.g., not more than twice per 24 hour period).

The mobile computing device 104 includes an application program 112 that retrieves pill release event data from the pill tracking dispenser 102, stores the pill release data on the mobile computing device 104, and transfers the pill release data to the mobile computing device 104 and/or computer system 106 for analysis. The application program 112 can be configured to provide the patient with information about the number of pills 108 released from the pill tracking dispenser 102 and whether the pills 108 were released in compliance with an established treatment protocol stored within the application program 112. The application program 112 can also be configured to alert the patient that it is time to dispense and ingest a pill 108 in accordance with a treatment protocol stored within the application program 112. The treatment protocol within the application program 112 can be remotely updated by a clinician using the networked connection between the computer system 106 and the mobile computing device 104. With this information, the application program 112 can be configured to provide treatment protocol compliance information to the patient through a visual interface on the mobile computing device 104.

In addition to exchanging compliance information between the pill tracking dispenser 102 and the computer system 106, the oral medication compliance system 100 can be configured to assist the patient with ordering refills for the oral medication 108. In some embodiments, the application program 112 is configured to advise the patient that it is time to refill the oral medication 108 available through the pill tracking dispenser 102. In other embodiments, the application program 112 is configured to automatically communicate with a clinician at the computer system 106 to request refill instructions and permissions. In yet other embodiments, the application program 112 and computer system 106 cooperate to automatically request and fulfill a refill of the oral medication 108 through an online pharmacy or other supplier.

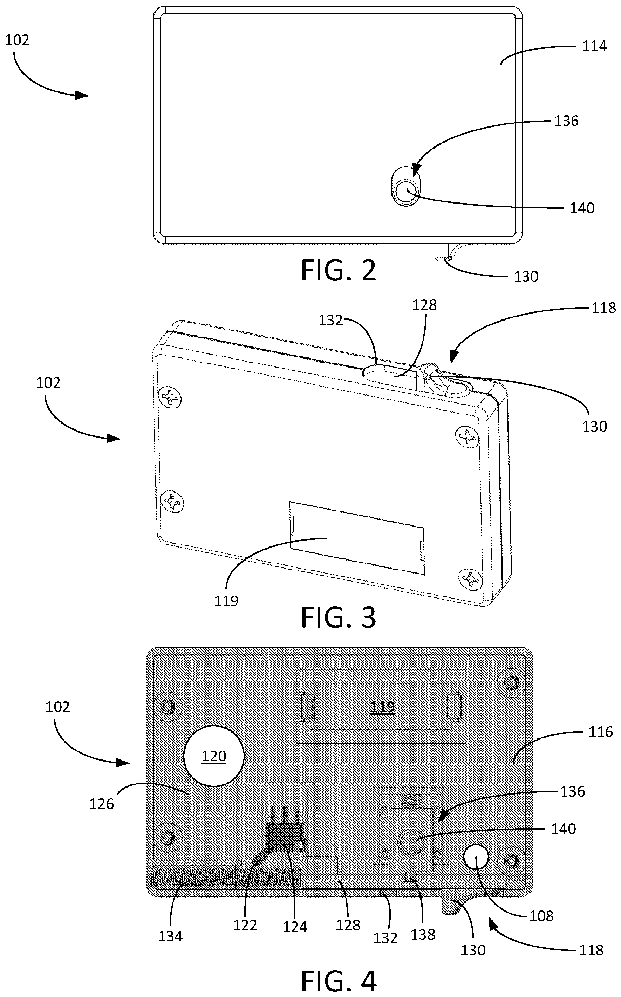

Turning to FIGS. 2-4, shown therein are general depictions of the pill tracking dispenser 102 constructed in accordance with a first embodiment. In this embodiment, the pill tracking dispenser 102 includes a housing 114, a pill chamber 116, a pill release mechanism 118, a removable pill refill door 119, a gate position sensor 122, an onboard processing unit 124 and an electronics chamber 126. In exemplary embodiments, the pill tracking dispenser 102 is manufactured from a lightweight, durable plastic and appropriately sized and configured for portable use. In some embodiments, the pill tracking dispenser 102 is configured to be easily carried on the patient's person. In the embodiment depicted in FIGS. 2-4, the pill tracking dispenser 102 is configured to be refillable by the patient by opening the pill refill door 119 to permit additional pills 108 to be loaded into the pill chamber 116.

The oral medication 108 is contained within the pill chamber 116 and released from the pill tracking dispenser 102 by actuating the pill release mechanism 118. In the embodiment depicted in FIGS. 2-4, the pill release mechanism 118 includes a gate 128 that can be manipulated between opened and closed positions using a trigger 130. When opened, the gate 128 reveals a pill port 132 in the housing 114 that permits the release of a pill 108 from the pill chamber 116. As depicted, a gate spring 134 applies a closing force to the gate 128 such that the gate 128 is biased in a closed position to prevent the unintentional release of pills 108 through the pill port 132. To prevent children from opening the gate 128 with the trigger 130, the pill release mechanism 118 includes a safety latch 136. The safety latch 136 includes a retractable, spring-loaded locking tab 138 that engages the gate 128 to prevent the gate 128 from opening while the locking tab 138 is deployed.

In embodiments that include the safety latch 136, the safety latch 136 must be retracted to withdraw the locking tab 138 from the gate 128 before the gate 128 can be opened with the trigger 130. A button 140 on the safety latch 136 can be used to manipulate the safety latch 136 between locked and unlocked positions. Thus, to release a pill 108, the patient must first unlock the safety latch 136 by pulling the button away from the gate 128 until the locking tab 138 is fully withdrawn from the gate 128. While holding the safety latch 136 in the unlocked position, the patient can then open the gate 128 by pressing down on the trigger 130 against the force of the gate spring 134. Once the trigger 130 and button 140 are released, the gate 128 closes and the safety latch 136 reengages in the locked position. In this way, two separate manipulations must be carried out to release a pill 108 from the pill tracking dispenser 102.

The onboard processing unit 124 includes a small microprocessor, digital memory, programming, an internal clock, and wireless network functionality (transmitter and receiver hardware). It will be appreciated that the onboard processing unit 124 may be a consolidated chip or a circuit board that includes a series of connected, but separate components. The onboard processing unit 124 can include an independent battery power source in addition to the primary battery 120. In some embodiments, the onboard processing unit 124 is configured to retrieve information from the mobile computing device 104 and to store that information within the pill tracking dispenser 102. For example, the onboard processing unit 124 can be configured to synchronize the internal clock within the onboard processing unit 124 with the date and time information on the mobile computing device 104.

The onboard processing unit 124 is operably connected to the gate position sensor 122. Each time the gate 128 is opened, the gate trips the gate position sensor 122, which is connected to the onboard processing unit 124. The gate position sensor 122 can be a mechanical switch that is closed (or opened) by interference with the moving gate 128. Each time the gate position sensor 122 is tripped, the onboard processing unit 124 records the gate opening event and correlates the event with the time the event took place. The record of each pill release event is stored within the onboard processing unit 124 and then automatically or manually transferred (uploaded) to the mobile computing device 104 when the pill tracking dispenser 102 is connected to the mobile computing device 104.

In some embodiments, the pill tracking dispenser 102 further includes a pill release sensor 142 that detects the actual movement of a pill 108 through the pill port 132. In some embodiments, the pill release sensor 142 is a small, low-power light curtain device that is activated when the gate position sensor 122 is triggered. Once activated, the pill release sensor 142 monitors the number of pills 108 that are discharged through the pill port 132 by detecting a sequence of breaks or interruptions in the light curtain. The output from the pill release sensor 142 is also provided to the onboard processing unit 124 so that the pill tracking dispenser 102 can track each gate opening event and the number of pills 108 dispensed while the gate 128 is in the open position. This prevents errors in compliance data that arise from multiple pills 108 being dispensed through a single gate opening event.

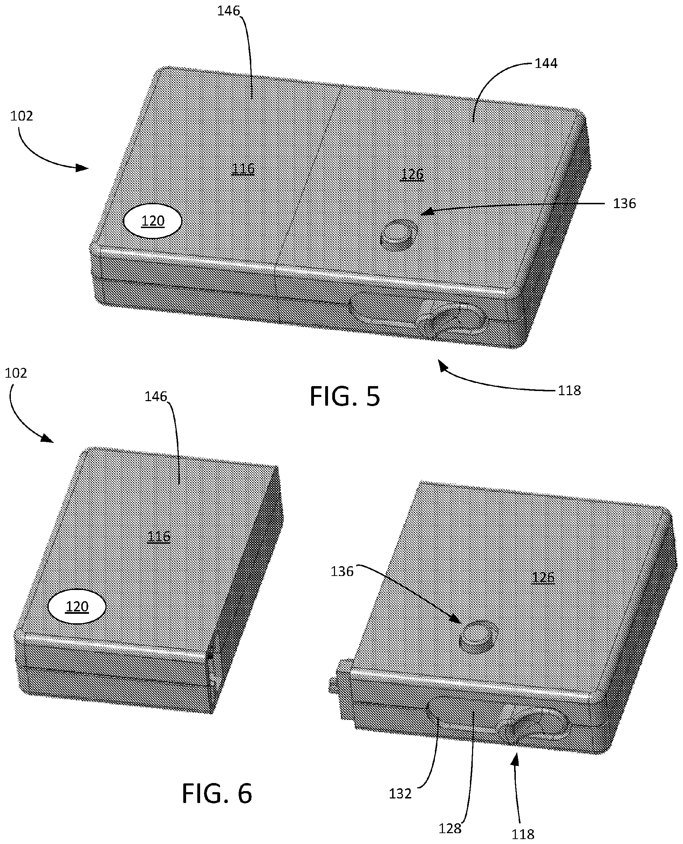

Turning to FIGS. 5 and 6, shown therein are top perspective views of the pill tracking dispenser 102 constructed in accordance with another embodiment. In this embodiment, the pill tracking dispenser 102 includes an upper module 144 and a lower module 146 that are configured for a mating connection. The upper module 144 includes the pill release mechanism 118, child safety latch 136, and a small time keeping battery integrated within the onboard processing unit 124. In this embodiment, the lower module 146 can be presented as a disposable unit that can be removed from the upper module 144 and discarded when the pill chamber 116 is empty. Although the onboard processing unit 124 is contained within the upper module 144, the primary battery 120 is placed in the lower module 146 and connected to the onboard processing unit 124 when the upper and lower modules 144, 146 are engaged. The battery 120 can be sized such that it is not configured to last longer than the number of pills 108 contained within the lower module 146. This permits the patient to keep the more expensive components of the pill tracking dispenser 102 found in the upper module 144 through multiple refills of the pills 108, and negates the need to charge the pill tracking dispenser 102 (i.e., the primary battery 120 in the lower module 146 powers the transmission of the dispensing data to the mobile computing device 104). In other embodiments, a rechargeable battery is placed in the upper module 144 or lower module 146 in place of the single-use battery 120.

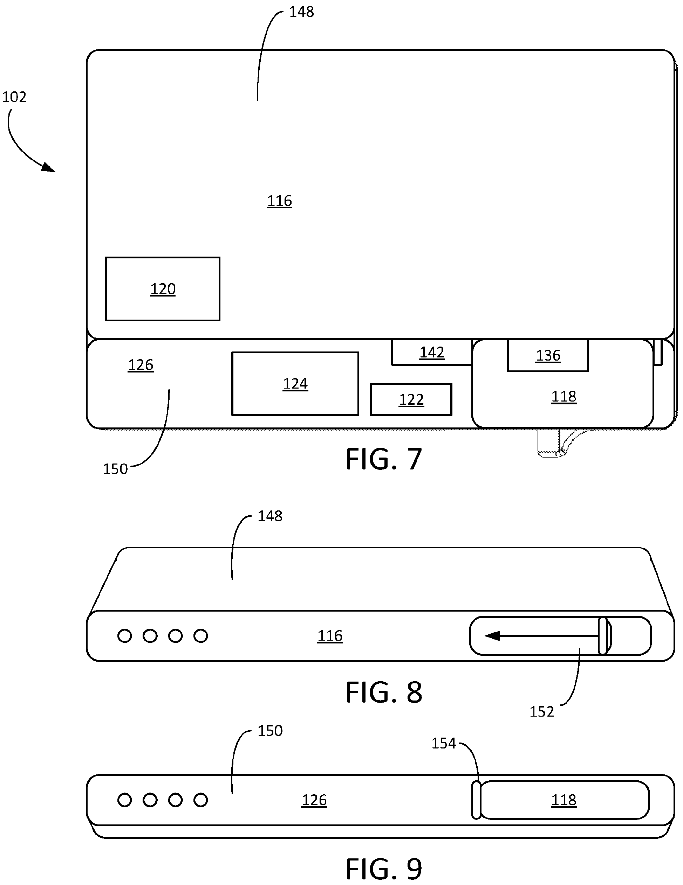

Turning to FIGS. 7-9, shown therein are depictions of another embodiment of the pill tracking dispenser 102 in which the pill chamber 116 is contained within a first side module 148 and the electronics chamber 126 and pill release mechanism 118 are contained within a second side module 150. The first side module 148 is designed to be a disposable unit that can be shipped to the patient with a refill of the medication 108. To contain the medication 108 inside the pill chamber 116 during shipping, the first side module 148 has a spring-loaded hatch 152 that is only opened when the first and second side modules 148, 150 are connected. The second side module 150 includes a protrusion 154 that engages the hatch 152 when the first and second side modules 148, 150 are engaged in a sliding motion that pulls the hatch 152 into an open position. Once fully engaged, the first and second side modules 148, 150 remain locked together with the internal hatch 152 open to permit the passage of pills 108 out of the pill chamber 116 and into the pill release mechanism 118. The second side module 150 can also include the safety latch 136 to prevent children from opening the pill release mechanism 118.

Thus, in the exemplary and non-limiting embodiments disclosed herein, an oral medication compliance system 100 includes a "smart" pill tracking dispenser 102 that is configured to track the release of pills 108 and provide that information to a patient, clinician, or study administrator through a networked connection that leverages the patient's mobile phone or other mobile computing device 104. The pill tracking dispenser 102 and oral medication compliance system 100 provide an effective tool for improving patient compliance with treatment protocols while also providing researchers or clinicians with valuable information about the correlation between patient outcomes and adherence to a treatment protocol.

It is to be understood that the terms "including", "comprising", "consisting" and grammatical variants thereof do not preclude the addition of one or more components, features, steps, or integers or groups thereof and that the terms are to be construed as specifying components, features, steps or integers. If the specification or claims refer to "an additional" element, that does not preclude there being more than one of the additional element. It is to be understood that where the claims or specification refer to "a" or "an" element, such reference is not be construed that there is only one of that element. It is to be understood that where the specification states that a component, feature, structure, or characteristic "may", "might", "can" or "could" be included, that particular component, feature, structure, or characteristic is not required to be included.

Where applicable, although state diagrams, flow diagrams or both may be used to describe embodiments, the present disclosure is not limited to those diagrams or to the corresponding descriptions. For example, flow need not move through each illustrated box or state, or in exactly the same order as illustrated and described. Methods of the present disclosure may be implemented by performing or completing manually, automatically, or a combination thereof, selected steps or tasks. The term "method" may refer to manners, means, techniques and procedures for accomplishing a given task including, but not limited to, those manners, means, techniques and procedures either known to, or readily developed from known manners, means, techniques and procedures by practitioners of the art to which the invention belongs.

For purposes of the instant disclosure, the term "at least" followed by a number is used herein to denote the start of a range beginning with that number (which may be a range having an upper limit or no upper limit, depending on the variable being defined). For example, "at least 1" means 1 or more than 1. The term "at most" followed by a number is used herein to denote the end of a range ending with that number (which may be a range having 1 or 0 as its lower limit, or a range having no lower limit, depending upon the variable being defined). For example, "at most 4" means 4 or less than 4, and "at most 40%" means 40% or less than 40%. Terms of approximation (e.g., "about", "substantially", "approximately", etc.) should be interpreted according to their ordinary and customary meanings as used in the associated art unless indicated otherwise. Absent a specific definition and absent ordinary and customary usage in the associated art, such terms should be interpreted to be at least ±20%, or at least ±10%, or at least ±5% of the base value.

When, in this document, a range is given as "(a first number) to (a second number)" or "(a first number) - (a second number)", this means a range whose lower limit is the first number and whose upper limit is the second number. For example, 25 to 100 should be interpreted to mean a range whose lower limit is 25 and whose upper limit is 100. Additionally, it should be noted that where a range is given, every possible subrange or interval within that range is also specifically intended unless the context indicates to the contrary. For example, if the specification indicates a range of 25 to 100 such range is also intended to include subranges such as 26-100, 27-100, etc., 25-99, 25-98, etc., as well as any other possible combination of lower and upper values within the stated range, e.g., 33-47, 60-97, 41-45, 28-96, etc. Note that integer range values have been used in this paragraph for purposes of illustration only and decimal and fractional values (e.g., 46.7-91.3) should also be understood to be intended as possible subrange endpoints unless specifically excluded. The range 0.5 mm to 10 mm is to be understood to include all integers within the range and decimal fractions within the range (e.g., 1, 2, 3, 4, 5, 6, 7, 8, 9, and 0.6, 0.7, 0.8, 0.9, 1.1, 1.2, 1.3, 1.4, 1.5, 1.6, 1.7, 1.8, 1.9, and 0.51, 0.52, 0.53, 0.54, 0.55, 0.56, 0.57, 0.58, 0.59, and so on).

It should be noted that where reference is made herein to a method comprising two or more defined steps, the defined steps can be carried out in any order or simultaneously (except where context excludes that possibility), and the method can also include one or more other steps which are carried out before any of the defined steps, between two of the defined steps, or after all of the defined steps (except where context excludes that possibility). Still further, additional aspects of the various embodiments of the instant disclosure may be found in one or more appendices attached hereto and/or filed herewith.

Thus, the embodiments of the present disclosure are well adapted to carry out the objects and attain the ends and advantages mentioned above as well as those inherent therein. While the inventive device and system have been described and illustrated herein by reference to particular non-limiting embodiments in relation to the drawings attached thereto, various changes and further modifications, apart from those shown or suggested herein, may be made therein by those of ordinary skill in the art.

## Claims

1. A system (100) for tracking patient compliance with a treatment protocol that includes periodic consumption of oral medication , the system comprising:
a mobile computing device (104) that includes an application program (112) configured to record the periodic consumption of oral medication by the patient; and
a pill tracking dispenser (102) that is configured for connection to the mobile computing device through a wireless network, wherein the pill tracking dispenser is configured to report to the mobile computing device the number of times the oral medication is consumed by the patient,
**characterized in that** the pill tracking dispenser comprises:
a pill chamber (116) configured to contain a plurality of pills (108); and
a pill release mechanism (118), wherein the pill release mechanism includes:
a gate (128) that permits a pill to be released from the pill chamber when the gate is opened; and
a trigger (130) connected to the gate, wherein the trigger can be manipulated by the patient to move the gate into an open position.

2. The system (100) of claim 1, wherein the system further comprises a clinician computer system (106) that is connected to the mobile computing device (104) through an internet connection.

3. The system (100) of claim 1, wherein the pill tracking dispenser (102) further comprises a safety latch mechanism (136) that is configured to prevent the unintentional release of medication from the pill chamber (116).

4. The system (100) of claim 3, wherein the safety latch mechanism (136) comprises:
a locking tab (138) that prevents the gate (128) from opening when the locking tab is in a deployed position; and
a button (140) connected to the locking tab, wherein the button can be manipulated by the patient to retract the locking tab from the deployed position to permit the gate to be opened by the patient.

5. The system (100) of claim 1, wherein the pill tracking dispenser (102) further comprises an electronics chamber (126) that includes an onboard processing unit (124), wherein the onboard processing unit is configured to establish a data connection with an oral medication compliance system that includes a mobile computing device (104) that is in turn configured for connection to a computer system (106).

6. The system (100) of claim 3, wherein the pill tracking dispenser (102) further comprises a gate position sensor (122), wherein the gate position sensor is configured to detect the gate (128) opening and provide the onboard processing unit (124) with a signal that the gate was opened to permit the release of a pill (108).

7. The system (100) of claim 3, wherein the pill tracking dispenser (102) further comprises a pill release sensor (142) that is configured to detect the release of a pill (108) from the pill chamber (116) through the gate (128).

8. The system (100) of claim 7, wherein the pill release sensor (142) comprises a light curtain that includes a light sensor and a light emitter that are activated when the gate (128) is opened.

9. The system (100) of claim 1, wherein the pill chamber (116) is contained within a first portion (148) of the pill tracking dispenser (102) and the electronics chamber (126) is contained within a second portion (150) of the pill tracking dispenser.

10. The system (100) of claim 9, wherein the first portion (148) of the pill tracking dispenser (102) is removable from the second portion (150) of the pill tracking dispenser.

11. The system (100) of claim 10, wherein the first portion (148) of the pill tracking dispenser (102) is configured as a disposable component that can be discarded when the pill chamber (116) is empty.

12. The system (100) of claim 1, wherein the application program (112) is also configured to advise the patient when it is time to consume the oral medication according to the treatment protocol.

13. A pill tracking dispenser (102), the pill tracking dispenser **characterized by**:
a pill chamber (116) configured to contain a plurality of pills (108);
a pill release mechanism (118), wherein the pill release mechanism includes a gate (128) that permits a pill to be released from the pill chamber when the gate is opened;
a trigger (130) connected to the gate, wherein the trigger can be manipulated by the patient to move the gate into an open position;
an electronics chamber (126) that includes an onboard processing unit (124), wherein the onboard processing unit is configured to establish a data connection with an oral medication compliance system (100) that includes a mobile computing device (104) that is in turn configured for connection to a computer system (106); and
a gate position sensor (122), wherein the gate position sensor is configured to detect the gate opening and provide the onboard processing unit with a signal that the gate was opened to permit the release of a pill
a safety latch mechanism (136) that is configured to prevent the unintentional release of medication from the pill chamber, wherein the safety latch mechanism comprises:
a locking tab (138) that prevents the gate from opening when the locking tab is in a deployed position; and
a button (140) connected to the locking tab, wherein the button can be manipulated by the patient to retract the locking tab from the deployed position to permit the gate to be opened by the patient.

## Patentansprüche

1. System (100) zur Überwachung der Einhaltung eines Behandlungsprotokolls durch einen Patienten, das die regelmäßige Einnahme oraler Medikamente enthält, wobei das System umfasst:
eine mobile Computervorrichtung (104), die ein Anwendungsprogramm (112) enthält, das so konfiguriert ist, dass es die regelmäßige Einnahme oraler Medikamente durch den Patienten aufzeichnet; sowie
einen Tablettenverfolgungsspender (102), der für die Verbindung mit der mobilen Computervorrichtung über ein drahtloses Netzwerk konfiguriert ist, wobei der Tablettenverfolgungsspender so konfiguriert ist, dass er der mobilen Computervorrichtung meldet, wie oft die oralen Medikamente vom Patienten eingenommen wurden,
**dadurch gekennzeichnet, dass** der Tablettenverfolgungsspender umfasst:
eine Tablettenkammer (116), die so konfiguriert ist, dass sie eine Vielzahl von Tabletten (108) enthält; und
einen Tablettenfreigabemechanismus (118), wobei der Tablettenfreigabemechanismus enthält:
eine Klappe (128), die es ermöglicht, dass eine Tablette aus der Tablettenkammer freigegeben wird, wenn die Klappe geöffnet wird; und
einen Auslöser (130), der mit der Klappe verbunden ist, wobei der Auslöser vom Patienten betätigt werden kann, um die Klappe in eine geöffnete Position zu bewegen.

2. System (100) nach Anspruch 1, wobei das System ferner ein klinisches Computersystem (106) umfasst, das über eine Internetverbindung mit der mobilen Computervorrichtung (104) verbunden ist.

3. System (100) nach Anspruch 1, wobei der Tablettenverfolgungsspender (102) ferner einen Sicherheitsverriegelungsmechanismus (136) umfasst, der so konfiguriert ist, dass er die unbeabsichtigte Freigabe von Medikamenten aus der Tablettenkammer (116) verhindert.

4. System (100) nach Anspruch 3, wobei der Sicherheitsverriegelungsmechanismus (136) umfasst:
eine Verriegelungslasche (138), die verhindert, dass sich die Klappe (128) öffnet, wenn sich die Verriegelungslasche in einer ausgefahrenen Position befindet; und
einen Knopf (140), der mit der Verriegelungslasche verbunden ist, wobei der Knopf vom Patienten betätigt werden kann, um die Verriegelungslasche aus der ausgefahrenen Position zurückzuziehen, damit die Klappe vom Patienten geöffnet werden kann.

5. System (100) nach Anspruch 1, wobei der Tablettenverfolgungsspender (102) ferner eine Elektronikkammer (126) umfasst, die eine integrierte Verarbeitungseinheit (124) enthält, wobei die integrierte Verarbeitungseinheit so konfiguriert ist, dass sie eine Datenverbindung mit einem System zur Überwachung der Einnahme oraler Medikamente herstellt, das eine mobile Computervorrichtung (104) enthält, die wiederum für die Verbindung mit einem Computersystem (106) konfiguriert ist.

6. System (100) nach Anspruch 3, wobei der Tablettenverfolgungsspender (102) ferner einen Klappenpositionssensor (122) umfasst, wobei der Klappenpositionssensor so konfiguriert ist, dass er das Öffnen der Klappe (128) erfasst und der integrierten Verarbeitungseinheit (124) ein Signal übermittelt, dass die Klappe geöffnet wurde, um die Freigabe einer Tablette (108) zu ermöglichen.

7. System (100) nach Anspruch 3, wobei der Tablettenverfolgungsspender (102) ferner einen Tablettenfreigabesensor (142) umfasst, der so konfiguriert ist, dass er die Freigabe einer Tablette (108) aus der Tablettenkammer (116) durch die Klappe (128) erfasst.

8. System (100) nach Anspruch 7, wobei der Tablettenfreigabesensor (142) einen Lichtvorhang umfasst, der einen Lichtsensor und einen Lichtemitter enthält, die aktiviert werden, wenn die Klappe (128) geöffnet wird.

9. System (100) nach Anspruch 1, wobei die Tablettenkammer (116) in einem ersten Abschnitt (148) des Tablettenverfolgungsspenders (102) enthalten ist und die Elektronikkammer (126) in einem zweiten Abschnitt (150) des Tablettenverfolgungsspenders enthalten ist.

10. System (100) nach Anspruch 9, wobei der erste Abschnitt (148) des Tablettenverfolgungsspenders (102) vom zweiten Abschnitt (150) des Tablettenverfolgungsspenders abnehmbar ist.

11. System (100) nach Anspruch 10, wobei der erste Abschnitt (148) des Tablettenverfolgungsspenders (102) als Einwegkomponente konfiguriert ist, die entsorgt werden kann, wenn die Tablettenkammer (116) leer ist.

12. System (100) nach Anspruch 1, wobei das Anwendungsprogramm (112) so konfiguriert ist, dass es den Patienten darauf hinweist, wann es Zeit ist, das orale Medikament gemäß dem Behandlungsprotokoll einzunehmen.

13. Tablettenverfolgungsspender (102), wobei der Tablettenverfolgungsspender **gekennzeichnet ist durch**:
eine Tablettenkammer (116), die so konfiguriert ist, dass sie eine Vielzahl von Tabletten (108) enthält;
einen Tablettenfreigabemechanismus (118), wobei der Tablettenfreigabemechanismus eine Klappe (128) enthält, die es ermöglicht, dass eine Tablette aus der Tablettenkammer freigegeben wird, wenn die Klappe geöffnet wird;
einen Auslöser (130), der mit der Klappe verbunden ist, wobei der Auslöser vom Patienten betätigt werden kann, um die Klappe in eine geöffnete Position zu bewegen;
eine Elektronikkammer (126), die eine integrierte Verarbeitungseinheit (124) enthält, wobei die integrierte Verarbeitungseinheit so konfiguriert ist, dass sie eine Datenverbindung mit einem System (100) zur Überwachung der Einnahme oraler Medikamente herstellt, das eine mobile Computervorrichtung (104) enthält, die wiederum für die Verbindung mit einem Computersystem (106) konfiguriert ist; und
einen Klappenpositionssensor (122), wobei der Klappenpositionssensor so konfiguriert ist, dass er das Öffnen der Klappe erfasst und der integrierten Verarbeitungseinheit ein Signal übermittelt, dass die Klappe geöffnet wurde, um die Freigabe einer Tablette zu ermöglichen,
einen Sicherheitsverriegelungsmechanismus (136), der so konfiguriert ist, dass er die unbeabsichtigte Freigabe von Medikamenten aus der Tablettenkammer verhindert, wobei der Sicherheitsverriegelungsmechanismus umfasst:
eine Verriegelungslasche (138), die verhindert, dass sich die Klappe öffnet, wenn sich die Verriegelungslasche in einer ausgefahrenen Position befindet; und
einen Knopf (140), der mit der Verriegelungslasche verbunden ist, wobei der Knopf vom Patienten betätigt werden kann, um die Verriegelungslasche aus der ausgefahrenen Position zurückzuziehen, damit die Klappe vom Patienten geöffnet werden kann.

## Revendications

1. Système (100) de suivi de l'observance du protocole de traitement par un patient, lequel protocole comprend la consommation périodique d'un médicament oral, le système comprenant :
un dispositif informatique mobile (104) qui comprend un programme d'application (112) configuré pour enregistrer la consommation périodique du médicament oral par le patient, et
un distributeur de suivi de pilules (102) qui est configuré pour une connexion au dispositif informatique mobile par l'intermédiaire d'un réseau sans fil, ledit distributeur de suivi de pilules étant configuré pour signaler au dispositif informatique mobile le nombre de fois où le médicament oral a été consommé par le patient ;
**caractérisé en ce que** le distributeur de suivi de pilules comprend :
un compartiment à pilules (116) configuré pour contenir une pluralité de pilules (108), et
un mécanisme de libération de pilule (118), ledit mécanisme de libération de pilule comprenant :
une porte (128) qui permet à une pilule d'être libérée du compartiment à pilules quand la porte est ouverte, et
un déclencheur (130) relié à la porte, ledit déclencheur pouvant être manipulé par le patient pour faire passer la porte en position ouverte.

2. Système (100) selon la revendication 1, comprenant en outre un système informatique du clinicien (106) qui est connecté au dispositif informatique mobile (104) par une connexion internet.

3. Système (100) selon la revendication 1, dans lequel le distributeur de suivi de pilules (102) comprend en outre un mécanisme de verrouillage de sécurité (136) qui est configuré pour empêcher la libération non intentionnelle de médicament par le compartiment à pilules (116).

4. Système (100) selon la revendication 3, dans lequel le mécanisme de verrouillage de sécurité (136) comprend :
une languette de verrouillage (138) qui empêche la porte (128) de s'ouvrir quand la languette de verrouillage est en position déployée, et
un bouton (140) relié à la languette de verrouillage, ledit bouton pouvant être manipulé par le patient pour rétracter la languette de verrouillage de sa position déployée afin de permettre l'ouverture de la porte par le patient.

5. Système (100) selon la revendication 1, dans lequel le distributeur de suivi de pilules (102) comprend en outre un compartiment électronique (126) qui comprend une unité de traitement intégrée (124), ladite unité de traitement intégrée étant configurée pour établir une connexion de données avec un système d'observance de traitement par médicament oral qui comprend un dispositif informatique mobile (104) qui est lui-même configuré pour une connexion à un système informatique (106).

6. Système (100) selon la revendication 3, dans lequel le distributeur de suivi de pilules (102) comprend en outre un capteur de position de porte (122), ledit capteur de position de porte étant configuré pour détecter l'ouverture de la porte (128) et pour fournir à l'unité de traitement intégrée (124) un signal indiquant que la porte a été ouverte pour permettre la libération d'une pilule (108).

7. Système (100) selon la revendication 3, dans lequel le distributeur de suivi de pilules (102) comprend en outre un capteur de libération de pilule (142) qui est configuré pour détecter la libération, par le compartiment à pilules (116), d'une pilule (108) sortant par la porte (128).

8. Système (100) selon la revendication 7, dans lequel le capteur de libération de pilule (142) comprend un rideau lumineux qui comprend un capteur de lumière et un émetteur de lumière qui sont activés quand la porte (128) est ouverte.

9. Système (100) selon la revendication 1, dans lequel le compartiment à pilules (116) est contenu dans une première partie (148) du distributeur de suivi de pilules (102) et le compartiment électronique (126) est contenu dans une deuxième partie (150) du distributeur de suivi de pilules.

10. Système (100) selon la revendication 9, dans lequel la première partie (148) du distributeur de suivi de pilules (102) est amovible par rapport à la deuxième partie (150) du distributeur de suivi de pilules.

11. Système (100) selon la revendication 10, dans lequel la première partie (148) du distributeur de suivi de pilules (102) est configurée comme un composant jetable qui peut être mis au rebut quand le compartiment à pilules (116) est vide.

12. Système (100) selon la revendication 1, dans lequel le programme d'application (112) est également configuré pour informer le patient du moment où il doit consommer le médicament oral conformément au protocole de traitement.

13. Distributeur de suivi de pilules (102), le distributeur de suivi de pilules étant **caractérisé par** :
un compartiment à pilules (116) configuré pour contenir une pluralité de pilules (108),
un mécanisme de libération de pilule (118), ledit mécanisme de libération de pilule comprenant une porte (128) qui permet à une pilule d'être libérée du compartiment à pilules quand la porte est ouverte,
un déclencheur (130) relié à la porte, ledit déclencheur pouvant être manipulé par le patient pour faire passer la porte en position ouverte,
un compartiment électronique (126) qui comprend une unité de traitement intégrée (124), ladite unité de traitement intégrée étant configurée pour établir une connexion de données avec un système d'observance de traitement par médicament oral (100) qui comprend un dispositif informatique mobile (104) qui est lui-même configuré pour une connexion à un système informatique (106),
un capteur de position de porte (122), ledit capteur de position de porte étant configuré pour détecter l'ouverture de la porte et pour fournir à l'unité de traitement intégrée un signal indiquant que la porte a été ouverte pour permettre la libération d'une pilule, et
un mécanisme de verrouillage de sécurité (136) qui est configuré pour empêcher la libération non intentionnelle de médicament par le compartiment à pilules, ledit mécanisme de verrouillage de sécurité comprenant :
une languette de verrouillage (138) qui empêche la porte de s'ouvrir quand la languette de verrouillage est en position déployée, et
un bouton (140) relié à la languette de verrouillage, ledit bouton pouvant être manipulé par le patient pour rétracter la languette de verrouillage de sa position déployée afin de permettre l'ouverture de la porte par le patient.
